# EUROPÄISCHE PATENTSCHRIFT

(11) **EP 0 535 353 B1**
(45) Veröffentlichungstag und Bekanntmachung des Hinweises auf die Patenterteilung: **25.01.1995**
(21) Anmeldenummer: 92114194.1
(22) Anmeldetag: 20.08.1992
(51) Int. Cl.: C07C 43/13, C07D 317/18, C07C 41/16

(54) **Verfahren zur Herstellung von Diglycerin und/oder Polyglycerin**
Process for the preparation of diglycerine and/or polyglycerine
Procédé pour la préparation de diglycérol et/ou polyglycérol

(30) Priorität: 27.09.1991 DE 4132171
(43) Veröffentlichungstag der Anmeldung: 07.04.1993
(73) Patentinhaber: DEUTSCHE SOLVAY-WERKE GMBH, 42697 Solingen (DE)
(72) Erfinder: Dillenburg, Helmut, Dr., W-4134 Rheinberg 1 (DE); Siemanowski, Werner, Dr., W-4134 Rheinberg (DE); Jakobson, Gerald, Dr., W-4134 Rheinberg 2 (DE)
(74) Vertreter: Lauer, Dieter, Dr.

(56) Entgegenhaltungen:
- EP-A- 0 296 341
- EP-A- 0 333 984
- EP-A- 0 374 699
- EP-A- 0 377 150
- US-A- 2 520 670
- CHEMICAL ABSTRACTS, vol. 113, no. 21, 19. November 1990, Columbus, Ohio, US; abstract no. 190747w, I. NARIBAYASHI ET AL 'Preparation of linear-chain high-purity diglycerin via diacetals.' Seite 663-664 ;
- CHEMICAL ABSTRACTS, vol. 114, no. 3, 21. Januar 1991, Columbus, Ohio, US; abstract no. 23414z, K. MATSUSHITA ET AL 'Preparation of linear diglycerin as surfactant.' Seite 654 ;Spalte 1 ;

## Beschreibung

Die vorliegende Erfindung betrifft ein Verfahren zur Herstellung von Diglycerin und/oder Polyglycerin mit einem sehr geringen Anteil an cyclischen Komponenten, wobei Isopropylidenglycerin mit α-Monochlorhydrin in Gegenwart mindestens einer alkalischen Verbindung bei bestimmten Temperaturen zu Monoisopropylidendiglycerin umgesetzt wird und nachfolgend das Monoisopropylidendiglycerin (gegebenenfalls mit Anteil von Monoisopropylidentriglycerin und/oder Monoisopropylidentetraglycerin) in Gegenwart mindestens eines sauren Katalysators und/oder eines sauren Ionenaustauschers sowie von Wasser bei bestimmten Temperaturen zu Diglycerin und/oder Triglycerin und/oder anderen Polyglycerinen und Aceton umgesetzt wird.

Aus Chemical Abstracts Vol. 113, Nr. 21, Abstract Nr. 190 747w ist ein Verfahren zur Herstellung hochreiner, linearkettiger Diglycerine bekannt, bei dem Glycerinacetale tropfenweise zu einer Mischung aus Epichlorhydrin, einer quartären Ammoniumverbindung und NaOH zugegeben wird. Der hierbei gebildete Isopropylidenglycidylether muß zur Hydrolyse der Epoxidfunktion nachfolgend zunächst mit Bortrifluorid-Diethylether-Komplex in Methylisobutylketon als Lösungsmittel und dann unter Wasserzugabe umgesetzt werden, wobei auch die Acetalgruppe abgespalten und das Diglycerinprodukt gebildet wird.

Aus der EP-A 296 341 ist ein Verfahren zur Herstellung von Polyglycerinen mit gegebenenfalls einem geringen Anteil an cyclischen Komponenten bekannt, bei dem Glycerin und/oder Diglycerin in der Wärme (340 bis 410 K) mit gasförmigem Chlorwasserstoff oder mit konzentrierter Salzsäure zu einem Gemisch aus α-Monochlorhydrin des Glycerins bzw. des Diglycerins und Glycerin bzw. Diglycerin umgesetzt wird; das entstandene Gemisch oder das daraus abgetrennte α-Monochlorhydrin wird nachfolgend bei Temperaturen von 320 bis 410 K zu Alkaliglycerinolat bzw. Alkalidiglycerinolat zugegeben, wonach man das gebildete Glycerin/- bzw. Diglycerin/Polyglycerin-Gemisch von den entstandenen Salzen befreit und das Polyglycerin abtrennt.

Aus der DE-OS 39 00 059 ist bereits ein Verfahren zur Herstellung von Polyglycerin bekannt, wobei α-Monochlorhydrin mit Epichlorhydrin bei Temperaturen von 20 bis 120 °C in einem Molverhältnis von Epichlorhydrin zu α-Monochlorhydrin von 0,8 : 1 bis 1 : 2,5 in Gegenwart von Säuren oder sauer reagierenden Verbindungen umgesetzt und das Reaktionsgemisch mit einem alkalisch reagierenden Medium umgesetzt wird und nach Wasserzugabe über einen oder mehreren Kationenaustauscher und nachfolgend unter Verwendung von Anionenaustauschern entsalzt und durch Destillation in Diglycerin, gegebenenfalls Polyglycerin, aufgetrennt wird.

Dieses Verfahren hat den Nachteil, daß bei der Umsetzung von α-Monochlorhydrin mit Epichlorhydrin die Anteile an Tri-, Tetraglycerin und höheren Polyglycerinen und der Anteil an cyclischen Komponenten relativ hoch sind und als Nebenprodukt eine chlorhaltige Verbindung auftritt, die schwer hydrolysierbar ist und das Polyglycerin verunreinigt.

Ziel und Aufgabe der vorliegenden Erfindung war es, ein verbessertes Verfahren zur Herstellung von Diglycerin und/oder Polyglycerin zu finden.

Insbesondere sollte der Anteil an cyclischen Komponenten verringert werden, Diglycerin als Hauptprodukt anfallen und die Bildung chlorhaltiger schwer hydrolysierbarer organischer Verbindungen weitgehend vermieden werden.

Erfindungsgemaß wurde festgestellt, daß diesen Zielen und Aufgaben ein Verfahren zur Herstellung von Diglycerin und/oder Polyglycerin mit einem sehr geringen Anteil an cyclischen Komponenten gerecht wird. Gemäß der Erfindung wird Isopropylidenglycerin mit mindestens einer alkalischen Verbindung, vorzugsweise Alkalilauge, vorgelegt und unter Zugabe von α-Monochlorhydrin bei Temperaturen von
10 bis 110 °C, vorzugsweise
40 bis 100 °C,
zu Monoisopropylidendiglycerin, gegebenenfalls mit einem Anteil von Monoisopropylidentriglycerin und/oder Monoisopropylidentetraglycerin umgesetzt, wobei (bezogen auf eine stöchiometrische Umsetzung) ein Überschuß an Isopropylidenglycerin eingesetzt wird. Aus dem Reaktionsgemisch werden die Hauptmenge des entstandenen Salzes oder der Salze und/oder das Wasser abgetrennt sowie das überschüssige Isopropylidenglycerin destillativ entfernt und nachfolgend wird das Monoisopropylidendiglycerin (gegebenenfalls mit einem Anteil von Monoisopropylidentriglycerin und/oder Monoisopropylidentetraglycerin) in Gegenwart mindestens eines sauren Katalysators und/oder eines sauren Ionenaustauschers sowie in Gegenwart von Wasser bei Temperaturen zwischen
20 und 110 °C, vorzugsweise
60 bis 100 °C,
zu Diglycerin und/oder Triglycerin und/oder anderen Polyglycerinen und Aceton umgesetzt.

Bei der Umsetzung von Isopropylidenglycerin mit α-Monochlorhydrin wird das Isopropylidenglycerin mit Alkalihydroxid vorgelegt und das α-Monochlorhydrin zugegeben, vorzugsweise kontinuierlich zugefügt. Wird in dieser Reihenfolge nicht gearbeitet, so besteht das Risiko, daß andere Verbindungen, insbesondere auch cyclische Verbindungen, entstehen.

Vorzugsweise wird das Isopropylidenglycerin mit α-Monochlorhydrin in Gegenwart von Alkalihydroxid, vorzugsweise Natriumhydroxid oder Natronlauge, in einem molaren Verhältnis von α-Monochlorhydrin zu Alkalihydroxid, vorzugsweise zu NaOH (berechnet als Feststoff) von
1 : 1,001 bis 1 : 1,5, vorzugsweise
1 : 1,005 bis 1 : 1,3,
umgesetzt. Durch die Einstellung dieses molaren Verhältnisses von α-Monochlorhydrin zu NaOH wird erreicht, daß die Reaktion beschleunigt und daß sie annähernd quantitativ erfolgt.

Nach einer bevorzugten Ausführungsform wird Isopropylidenglycerin mit α-Monochlorhydrin in einem Molverhältnis von
1,001 : 1 bis 20 : 1, vorzugsweise
4 : 1 bis 10 : 1,
umgesetzt. Durch die Einhaltung dieses Molverhältnisses gelingt es, den Gehalt an cyclischen Verbindungen noch weiter zu verringern und das Verhältnis Diglycerin zu Polyglycerin insofern zu verschieben, daß der Anteil von Diglycerin vergrößert wird.

Nach erfolgter Umsetzung zu Monoisopropylidendiglycerin und nach destillativer Entfernung von Wasser wird das ausgefallene Salz vorzugsweise durch Filtration oder Zentrifugation abgetrennt. Wird während der Umsetzung die Wasserkomponente abdestilliert, so entsteht ein sehr schwer zu filtrierendes oder zu trennendes Salz.

Nach einer bevorzugten Ausführungsform wird nach Filtration des Salzes, vorzugsweise des Natriumchlorides, das im Überschuß eingesetzte Isopropylidenglycerin destillativ abgetrennt und im Kreislauf zur Umsetzung zurückgeführt.

Nach einer weiteren bevorzugten Ausführungsform wird das Aceton, das bei der sauer katalysierten wäßrigen Hydrolyse von Monoisopropylidendiglycerin anfällt, im Kreislauf geführt und zur Herstellung von Isopropylidenglycerin aus Glycerin und Aceton mitverwendet. Durch diese Verfahrensmaßnahmen gelingt eine Optimierung des Verfahrens.

Nach einer bevorzugten Ausführungsform wird das nach der sauer katalysierten Ketalspaltung in wäßriger Lösung entstandene und vom Aceton durch Abdestillation befreite Di- und/oder Polyglycerin, das einen Anteil von Glycerin enthält (entstanden aus nicht vollständig abdestillertem Isopropylidenglycerin in der Hydrolyse), durch eine Kombination mindestens eines Kationenaustauschers und nachfolgend mindestens eines Anionenaustauschers von dem gelösten Restgehalt des Salzes, vorzugsweise Natriumchlorides, befreit und nachfolgend durch fraktionierte Destillation in Wasser, Glycerin, Diglycerin und Polyglycerin aufgetrennt.

Nach einer weiteren Ausführungsform wird das Polyglycerin durch fraktionierte Destillation in Tri-, Tetra- und/oder höhere Polyglycerine aufgetrennt.

### Ausführungsbeispiele:

### 1. Ausführungsbeispiel

In einem 1-l-Doppelwandreaktor mit Rührer und Destillationsaufsatz werden 2 mol (264 g) Isopropylidenglycerin und 1,1 mol Natriumhydroxid (als 50 %ige wäßrige Lösung) vorgelegt und auf ca. 90 °C erwärmt. Bei dieser Temperatur werden innerhalb von 2 Stunden 1 mol (110,5 g) α-Monochlorhydrin unter Rühren zudosiert. Nach einer halbstündigen Nachreaktionszeit wird das im Reaktionsgemisch vorhandene Wasser im Vakuum abdestilliert und anschließend das ausgefallene Salz abfiltriert.

Das Filtrat wird in den Reaktor zurückgeführt und das im Überschuß eingesetzte Isopropylidenglycerin bei 120 °C Heizöltemperatur (Ölbadtemperatur) und 20 mbar abdestilliert.

Der Rückstand im Reaktor wird mit dem gleichen Volumenanteil an destilliertem Wasser versetzt, mit konzentrierter HCl-Lösung (30 gew.-%ig) angesäuert und bei 100 °C Heizöltemperatur (Ölbadtemperatur) entstehendes Aceton abdestilliert (Dauer der Ketalspaltung ca. 0,5 Stunden).

Die wäßrige Roh-Diglycerinlösung wird über eine Kombination von Kationen- und Anionenaustauscher entsalzt und abschließend im Vakuum eingedampft. Als Rohprodukt fallen ca. 126 g an.

| GC-Analyse des Rohprodukts (Angaben in g/kg) | |
|---|---|
| Glycerin | 160,2 |
| cyclisches Diglycerin | 2,5 |
| Diglycerin | 608,6 |
| cyclisches Triglycerin | 1,2 |
| Triglycerin | 162,3 |
| cyclisches Tetraglycerin | 1,2 |
| Tetraglycerin | 46,3 |
| cyclisches Pentaglycerin | 4,9 |
| Pentaglycerin | 8,4 |
| Hexaglycerin | 3,3 |
| Heptaglycerin | 1,0 |

### 2. Ausführungsbeispiel

- Einsatzstoffe:: 6 mol (793 g) Isopropylidenglycerin
1,025 mol ( 41 g) NaOH (als 50 %ige wäßrige Lösung eingesetzt)
1 mol (110,5 g) α-Monochlorhydrin

Durchführung der Synthese wie im 1. Ausführungsbeispiel, jedoch in der 1. Synthesestufe bei 30 °C; Ausbeute an Rohprodukt: 135 g

| GC-Analyse des Rohprodukts (Angaben in g/kg) | |
|---|---|
| Glycerin | 78,5 |
| cyclisches Diglycerin | 18,7 |
| Diglycerin | 717,3 |
| cyclisches Triglycerin | 6,2 |
| Triglycerin | 133,8 |
| cyclisches Tetraglycerin | 1,7 |
| Tetraglycerin | 31,7 |
| cyclisches Pentaglycerin | 1,0 |
| Pentaglycerin | 8,3 |
| Hexaglycerin | 2,3 |

### 3. Ausführungsbeispiel

- Einsatzstoffe:: 8 mol (1056 g) Isopropylidenglycerin
1,1 mol ( 44 g) NaOH (als 50 %ige wäßrige Lösung eingesetzt)
1 mol (110,5 g) α-Monochlorhydrin

Durchführung der Synthese wie im 1. Ausführungsbeispiel, bei 90 °C in der 1. Synthesestufe
Ausbeute an Rohprodukt: 147 g

| GC-Analyse des Rohprodukts (Angaben in g/kg) | |
|---|---|
| Glycerin | 75,0 |
| cyclisches Diglycerin | 1,2 |
| Diglycerin | 815,8 |
| cyclisches Triglycerin | 1,2 |
| Triglycerin | 90,4 |
| Tetraglycerin | 13,6 |
| Pentaglycerin | 1,7 |

Die Rohprodukte der Ausführungsbeispiele 1 bis 3 wurden einer fraktionierten Destillation unterworfen und dabei in Glycerin, Diglycerin und Polyglycerin aufgetrennt.

## Patentansprüche

1. Verfahren zur Herstellung von Diglycerin und/oder Polyglycerin mit einem sehr geringen Anteil an cyclischen Komponenten, dadurch gekennzeichnet, daß Isopropylidenglycerin mit mindestens einer alkalischen Verbindung, vorzugsweise Alkalilauge, vorgelegt und unter Zugabe von α-Monochlorhydrin bei Temperaturen von
10 bis 110°C, vorzugsweise
40 bis 100°C,
zu Monoisopropylidendiglycerin, gegebenenfalls mit einem Anteil von Monoisopropylidentriglycerin und/oder Monoisopropylidentetraglycerin, umgesetzt wird, wobei (bezogen auf eine stöchiometrische Umsetzung) ein Überschuß an Isopropylidenglycerin eingesetzt wird, daß aus dem Reaktionsgemisch die Hauptmenge des entstandenen Salzes und/oder das Wasser abgetrennt sowie das überschüssige Isopropylidenglycerin destillativ entfernt wird und nachfolgend das Monoisopropylidendiglycerin (gegebenenfalls mit einem Anteil von Monoisopropylidentriglycerin und/oder Monoisopropylidentetraglycerin) in Gegenwart mindestens eines sauren Katalysators und/oder eines sauren Ionenaustauschers sowie in Gegenwart von Wasser bei Temperaturen zwischen
20 und 110 °C, vorzugsweise
60 bis 100 °C,
zu Diglycerin und/oder Triglycerin und/oder anderen Polyglycerinen und Aceton umgesetzt wird.

2. Verfahren nach Anspruch 1, dadurch gekennzeichnet, daß Isopropylidenglycerin mit α-Monochlorhydrin in Gegenwart von Alkalihydroxid, vorzugsweise Natriumhydroxid oder Natronlauge, in einem molaren Verhältnis von α-Monochlorhydrin zu Alkalihydroxid, vorzugsweise NaOH (berechnet als Feststoff; wasserfrei) von
1 : 1,001 bis 1 : 1,5, vorzugsweise
1 : 1,005 bis 1 : 1,3,
umgesetzt wird.

3. Verfahren nach Ansprüchen 1 und 2, dadurch gekennzeichnet, daß das α-Monochlorhydrin kontinuierlich zugefügt wird.

4. Verfahren nach einem oder mehreren der Ansprüche 1 bis 3, dadurch gekennzeichnet, daß Isopropylidenglycerin mit α-Monochlorhydrin in einem Molverhältnis von
1,001 : 1 bis 20 : 1, vorzugsweise
4 : 1 bis 10 : 1,
umgesetzt wird.

5. Verfahren nach einem oder mehreren der Ansprüche 1 bis 4, dadurch gekennzeichnet, daß nach erfolgter Umsetzung zu Monoisopropylidendiglycerin und nach destillativer Entfernung von Wasser das ausgefallene Salz vorzugsweise durch Filtration oder Zentrifugation abgetrennt wird.

6. Verfahren nach einem oder mehreren der Ansprüche 1 bis 5, dadurch gekennzeichnet, daß nach Filtration des Salzes, vorzugsweise des Natriumchlorides, das im Überschuß eingesetzte Isopropylidenglycerin destillativ abgetrennt und im Kreislauf zur Umsetzung zurückgeführt wird.

7. Verfahren nach einem oder mehreren der Ansprüche 1 bis 6, dadurch gekennzeichnet, daß das Aceton, das bei der sauer katalysierten wäßrigen Hydrolyse von Monoisopropylidendiglycerin anfällt, im Kreislauf geführt und zur Herstellung von Isopropylidenglycerin aus Glycerin und Aceton mitverwendet wird.

8. Verfahren nach einem oder mehreren der Ansprüche 1 bis 7, dadurch gekennzeichnet, daß das nach der sauer katalysierten Ketalspaltung in wäßriger Lösung entstandene und vom Aceton durch Abdestillation befreite Di- und/oder Polyglycerin, das einen Anteil von Glycerin enthält (entstanden aus nicht vollständig abdestilliertem Isopropylidenglycerin in der Hydrolyse), durch eine Kombination mindestens eines Kationenaustauschers und nachfolgend mindestens eines Anionenaustauschers von dem gelösten Restgehalt des Salzes, vorzugsweise Natriumchlorides, befreit und nachfolgend durch fraktionierte Destillation in Wasser, Glycerin, Diglycerin und Polyglycerin aufgetrennt wird.

9. Verfahren nach einem oder mehreren der Ansprüche 1 bis 8, dadurch gekennzeichnet, daß das Verfahren kontinuierlich durchgeführt wird.

## Claims

1. A method for preparing diglycerol and/or polyglycerol with a very small content of cyclic constituents, characterised in that isopropylidene glycerol is combined with at least one alkaline compound, preferably lye, and is reacted with the addition of α-monochlorohydrin at temperatures of
10 to 110°C, preferably
40 to 100°C,
to form monoisopropylidene diglycerol, optionally with a content of monoisopropylidene triglycerol and/or monoisopropylidene tetraglycerol, with (relative to a stoichiometric reaction) an excess of isopropylidene glycerol being used, that the majority of the resulting salt and/or the water is separated off from the reaction mixture and the excess isopropylidene glycerol is removed by distillation and subsequently the monoisopropylidene diglycerol (optionally with a content of monoisopropylidene triglycerol and/or monoisopropylidene tetraglycerol) is reacted in the presence of at least one acidic catalyst and/or an acidic ion exchanger and in the presence of water at temperatures between
20 and 110°C, preferably
60 to 100°C,
to form diglycerol and/or triglycerol and/or other polyglycerols and acetone.

2. A method according to Claim 1, characterised in that isopropylidene glycerol is reacted with α-monochlorohydrin in the presence of alkali hydroxide, preferably sodium hydroxide or sodium hydroxide solution, in a molar ratio of α-monochlorohydrin to alkali hydroxide, preferably NaOH (calculated as solid; anhydrous) of
1 : 1.001 to 1 : 1.5, preferably
1 : 1.005 to 1 : 1.3.

3. A method according to Claims 1 and 2, characterised in that the α-monochlorohydrin is added continuously.

4. A method according to one or more of Claims 1 to 3, characterised in that isopropylidene glycerol is reacted with α-monochlorohydrin in a molar ratio of
1.001 : 1 to 20 : 1, preferably
4 : 1 to 10 : 1.

5. A method according to one or more of Claims 1 to 4, characterised in that once reaction to monoisopropylidene diglycerol has taken place and once water has been removed by distillation, the resulting salt is separated off, preferably by filtration or centrifugation.

6. A method according to one or more of Claims 1 to 5, characterised in that after filtration of the salt, preferably of the sodium chloride, the isopropylidene glycerol used in excess is separated off by distillation and is recycled to the reaction.

7. A method according to one or more of Claims 1 to 6, characterised in that the acetone which is produced upon the acid-catalysed aqueous hydrolysis of monoisopropylidene diglycerol is recycled and is jointly used to produce isopropylidene glycerol from glycerol and acetone.

8. A method according to one or more of Claims 1 to 7, characterised in that the diglycerol and/or polyglycerol which is produced after the acid-catalysed ketal cleavage in aqueous solution and has been freed of acetone by distilling off, which contains a portion of glycerol (produced from isopropylidene glycerol which has been incompletely distilled off in the hydrolysis), is freed of the dissolved residual content of the salt, preferably sodium chloride, by a combination of at least one cation exchanger and subsequently at least one anion exchanger, and is subsequently separated by fractional distillation into water, glycerol, diglycerol and polyglycerol.

9. A method according to one or more of Claims 1 to 8, characterised in that the method is performed continuously.

## Revendications

1. Procédé de préparation de diglycérol et/ou de polyglycérol comportant une très faible proportion de constituants cycliques, procédé caractérisé en ce qu'on place tout d'abord l'isopropylidène glycérol avec au moins un composé alcalin, avantageusement de la lessive alcaline, et l'on fait réagir, avec addition d'alpha-monochlorhydrine à des températures de :
10 à 110°C , avantageusement
de 40 à 100°C,
pour obtenir du monoisopropylidène diglycérol, éventuellemment avec une proportion de monoisopropylidène triglycérol et/ou de monoisopropylidène tétraglycérol, en utilisant (par rapport à une réaction stoéchiométrique) un excès d'isopropylidène glycérol; en ce qu'on sépare du mélange réactionnel la majeure partie du sel formé et/ou de l'eau, que l'on élimine par distillation l'isopropylidène glycérol excédentaire et l'on fait ensuite réagir le monoisopropylidène diglycérol (comportant éventuellement une certaine proportion de monoisopropylidène triglécyrol et/ou de monoisopropylidène tétraglycérol), en présence d'au moins un catalyseur acide et/ou d'un échangeur acide d'ions, ainsi qu'en présence d'eau, à des températures comprises entre :
20°C et 110°C, avantageusement entre
60 et 100°C,
pour obtenir du diglycérol et/ou du triglycérol et/ou d'autres polyglycérols et de l'acétone.

2. Procédé selon la revendicatiobn 1, caractérisé en ce qu'on fait réagir l'isopropylidène glycérol avec l'alpha-monochlorhydrine en présence d'un hydroxyde alcalin, avantageusement l'hydroxyde de sodium ou de la lessive de soude, selon un rapport molaire entre l'alpha-monochlorhydrine et l'hydroxyde alcalin, avantageusement NaOH (calculé en solides, sans eau) de :
1 : 1,001 à 1 : 1,5, avantageusement
de 1 : 1,005 à 1 : 1,3.

3. Procédé selon les revendications 1 et 2, caractérisé en ce qu'on introduit en continu l'alpha-monochlorhydrine.

4. Procédé selon une ou plusieurs des revendications 1 à 3, caractérisé en ce qu'on fait réagir l'isopropylidène glycérol avec l'alpha-monochlorhydrine selon un rapport molaire de :
1,001 : 1 à 20 : 1, avantageusement
de 4 : 1 à 10 : 1.

5. Procédé selon une ou plusieurs des revendications 1 à 4, caractérisé en ce que, après réalisation de la réaction donnant du monoisopropylidène diglycérol et après avoir éliminé par distillation l'eau, on sépare, avantageusement par filtration ou par centrifugation, le sel précipité.

6. Procédé selon une ou plusieurs des revendications 1 à 5, caractérisé en ce qu'après filtration du sel, avantageusement du chlorure de sodium, on sépare par distillation l'isopropylidène glycérol utilisé en excès et on le fait revenir dans le circuit de la réaction.

7. Procédé selon une ou plusieurs des revendications 1 à 6, caractérisé en ce qu'on recycle l'acétone, que l'on obtient lors de l'hydrolyse aqueuse, catalysée par un acide, du monoisopropylidène diglycérol et on la co-utilise pour préparer l'isopropylidène glycérol à partir de glycérol et d'acétone.

8. Procédé selon une ou plusieurs des revendications 1 à 7, caractérisé en ce qu'on soumet le diglycérol et/ou le polyglycérol, résultant en solution aqueuse de la scission de cétal catalysée par un acide et dont on a éliminé l'acétone par distillation, et qui contient une certaine proportion de glycérol (résultant de l'isopropylidène glycérol qui n'a pas été entièrement chassé par distillation lors de l'hydrolyse) à une élimination de la teneur résiduelle en sel dissout, avantageusement le chlorure de sodium, élimination réalisée par une combinaison d'au moins un échangeur de cations et ensuite au moins un échangeur d'anions, et à une séparation subséquente, par distillation fractionnée, donnant de l'eau, du glycérol, du diglycérol et du polyglycérol.

9. Procédé selon une ou plusieurs des revendications 1 à 8, caractérisé en ce que le procédé est réalisé en continu.
